# EUROPÄISCHE PATENTANMELDUNG

(11) **EP 1 882 948 A2**
(43) Veröffentlichungstag der Anmeldung: **30.01.2008**
(21) Anmeldenummer: 06015812.8
(22) Anmeldetag: 28.07.2006
(51) Int. Cl.: G01N 35/00

(54) **Vorrichtung zur Probenverarbeitung**

(71) Anmelder: Qiagen GmbH, 40724 Hilden (DE); Qiagen Instruments AG, 8634 Hombrechtikon (CH)
(72) Erfinder: Alfredsson, Magnus, 8570 Weinfelden (CH); Lutze, Konstantin, 8708 Männedorf (CH); Deutschmann, Thomas, 42349 Wuppertal (DE); Aeschbacher, Jürg, 8634 Hombrechtikon (CH); Walder, Bruno, 8616 Riedikon (CH); Widler, Patrick, 8807 Freienbach (CH); Farner, Samuel, 8342 Wernetshausen (CH); Friederichs, Ulf, 8712 Stäfa (CH); Bützer, Axel, 8713 Uerikon (CH)
(74) Vertreter: Leidescher, Thomas

(57) **Zusammenfassung**

Vorrichtung zur Probenverarbeitung, insbesondere Probenaufbereitung sowie zur Vorbereitung und/oder gegebenenfalls zur Durchführung eines Folgeverfahrens für einen Analyten aus einer biologischen Probe, die ein Modul zur Aufnahme und/oder Ausgabe zumindest eines Proben- bzw. Prozessgefäßes, ein Modul zum Transport eines Prozessgefäßes, ein Modul zur Probenaufbereitung und ein Modul zur Einleitung eines Folgeverfahrens für einen Analyten umfasst. Die Module sind zu mindestens zwei Einheiten (120,140) zusammengefasst, die mindestens eine Steuerung mit Bedienoberfläche (590) besitzen und durch einen ersten Datenbus verbunden sind.

## Beschreibung

Die vorliegende Erfindung betrifft eine Vorrichtung zur Verarbeitung biologischer Proben, beispielsweise für die anschließende Analyse eines Biomoleküls, insbesondere einer Nukleinsäure oder eines Proteins, in dieser Probe. Die Vorrichtung ist zum Beispiel für Anwendungszwecke in der Biochemie, Molekulargenetik, Mikrobiologie, medizinischen Diagnostik oder forensischen Medizin geeignet.

Auf vielen technischen Gebieten wie beispielsweise der Chemie, der Biologie, der Medizin oder der Umwelttechnologie ist es notwendig, biologische Materialien (z.B. Flüssigkeiten) zu analysieren, zu verarbeiten oder miteinander in Reaktion zu bringen. Zu diesem Zweck werden die Flüssigkeiten oder Materialien mit verschiedenen Verfahren gefiltert, abgekühlt, erhitzt, in Bestandteile zerlegt, gewaschen, pipettiert oder mittels anderer Verfahren behandelt. Häufig ist es notwendig, eine lange und komplexe Folge von Verarbeitungsschritten zu durchlaufen, um das biologische Material zu präparieren. Weiterhin muss in vielen Fällen eine große Anzahl unterschiedlicher Materialien entsprechend der gleichen Sequenz prozessiert werden, oder es müssen Reihen der gleichen Materialien parallel prozessiert werden.

Dabei erlangen Verfahren im Bereich der Probenvorbereitung für Präparationen und/oder diagnostische oder analytische Untersuchungen, die insbesondere die Isolierung von Nukleinsäuren und/oder Proteinen als Verfahrensschritt enthalten, zunehmende Bedeutung. Dies gilt insbesondere für automatisierte Verfahren, da auf diese Weise eine große Anzahl von Proben innerhalb kurzer Zeit vorbereitet werden können. Dadurch werden die Voraussetzungen für ein effizientes Screening und/oder Analyse mit mittlerem bis hohem Probendurchsatz geschaffen. Dies ist von enormer Bedeutung, da eine rein manuelle Handhabung von sehr großen Probenzahlen praktisch schwer zu bewältigen und kostenintensiv ist.

Eine weit bekannte Methode des Reinigens der Biomoleküle basiert auf den Schritten des Aufschließens der Zellbestandteile einer biologischen Probe ("Lyse"), des selektiven Bindens eines oder mehrerer bestimmter Bestandteile aus dem erhaltenen Lysat an ein festes Stütz- oder Trägermaterial ("Binden"), des Beseitigens unerwünschter Bestandteile vom festen Stütz- oder Trägermaterial ("Waschen"), und des Ablösens/Lösens des gewünschten Bestandteils ("Elution").

Um ein gewünschtes Adsorbieren und Desorbieren während der Aufreinigung der Biomoleküle zuzulassen, sind spezielle Adsorptionsmaterialien entwickelt worden, die z.B. aus Siliziumoxid-haltigen Materialien wie aus Silica-Gel gebildet sind, und die meist als Partikel oder Filterelement vorliegen. Die Materialien weisen eine Oberfläche auf, an die die zu isolierenden Biomoleküle oder die abzutrennenden, nicht gewünschten Komponenten in einem spezifischen oder unspezifischen Prozess binden. Alternativ zur Adsorption an die Matrix werden in anderen Reinigungsverfahren Biomoleküle auf Filterelementen einfach durch den Effekt des Größenausschlusses zurückgehalten. Wenn eine Flüssigkeit, die ein Biomolekül wie z.B. eine Nukleinsäure enthält, durch das Filterelement hindurch tritt, bleiben Biomoleküle ab einer spezifischen Größe oder ein Teil davon in dem Filterelement, während der Rest durch das Filterelement hindurchgelangt. Die Trennleistung ist in diesem Fall u.a. von der Größe des Analyten abhängig und häufig können kleine Analyten auf diese Weise nicht separiert werden.

Ein weiteres bekanntes Verfahren, für das die Analytgröße von geringerer Bedeutung ist, betrifft die Zugabe von vorzugsweise magnetischen oder magnetisierbaren Partikeln, die eine Nukleinsäure- oder Protein-adsorbierende Substanz an ihrer Oberfläche aufweisen, und die anschließende Trennung der Partikel vom Rest der Probe vorzugsweise mittels magnetischer Trennverfahren. Bei Bindung des zu isolierenden Biomoleküls an eine derartige Partikel- oder Filteroberfläche wird zu dessen Rückgewinnung eine Elutionsflüssigkeit, z.B. Nuklease-freies Wasser, mit der Oberfläche in Kontakt gebracht. Auf diese Weise wird das gewünschte Biomolekül von der Adsorptionsmatrix desorbiert bzw. gelöst (eluiert) und in einem Gefäß aufgefangen.

Es ist bekannt, die oben angeführten Verfahrensschritte in einer einzigen automatisierten Vorrichtung durchführen zu lassen. Die zu analysierenden Proben - wie etwa Blut oder Urin - werden dazu entweder von der Vorrichtung oder vor der Aufbringung auf die Vorrichtung üblicherweise in ein oder mehrere Prozessgefäße überführt, die dann in der Vorrichtung einer definierten Reihe von Arbeitsschritten unterzogen werden. Die Vorrichtung liefert üblicherweise einen Träger mit einer Mehrzahl von Vertiefungen oder Gefäßen, in denen die aufgereinigten Proben ausgegeben werden.

Das Dokument WO9916781 beschreibt ein Verfahren zur Isolierung eines Analyten aus einer biologischen Probe. Es umfasst die Schritte des Aufschließens der Probe in einem Reaktionsgefäß, des Zugebens einer festen Adsorptionsmatrix, des Inkubierens unter Bedingungen, bei denen der Analyt an die Adsorptionsmatrix bindet, des Entfernens nichtgebundener Probenbestandteile aus dem Reaktionsgefäß, des Inkubierens unter Bedingungen, bei denen der Analyt von der Adsorptionsmatrix eluiert wird, und des Abtrennens des Eluats von der Adsorptionsmatrix. Ferner wird eine entsprechende Vorrichtung zur Isolierung eines Analyten aus einer biologischen Probe beschrieben, die einen Probenvorbereiter, eine Aufnahmeeinrichtung für Reagenzien, eine erste Aufnahmeeinrichtung für Reaktionsgefäße zur Probenvorbereitung, eine zweite Aufnahmeeinrichtung für Reaktionsgefäße sowie ein Robotik-Werkzeugmittel umfasst.

Obwohl der wesentliche Aufbau von automatischen Probenvorbereitern bekannt ist, lässt der Stand der Technik großen Spielraum für Verbesserungen. Es ist daher Aufgabe der vorliegenden Erfindung, einen Probenvorbereiter im Hinblick auf eine Steigerung der Wirtschaftlichkeit und Wartungsfreundlichkeit sowie auf vereinfachte Bedienung und damit eine geringere Häufigkeit von Fehlbedienungen auszugestalten. Dem Anwender soll zudem ein hohes Maß an Flexibilität und Variabilität ermöglicht werden sowie die Voraussetzungen geschaffen werden, um eine durchgehende Prozesskette ablaufen zu lassen, die kein weiteres Eingreifen und keine weitere Kontrolle seitens des Bedieners nach dem Start erfordert.

Diese Aufgabe wird gelöst durch eine Vorrichtung gemäß den Ansprüchen 1 beziehungsweise 2, eine Einheit gemäß Anspruch 9 und ein Modul nach Anspruch 15. Weitere Einzelheiten, Vorteile und Aspekte der vorliegenden Erfindung ergeben sich aus den Unteransprüchen, der Beschreibung sowie den beigefügten Zeichnungen.

Typischerweise umfasst die erfindungsgemäße Vorrichtung zur Probenverarbeitung, insbesondere zur Probenaufbereitung sowie zur Vorbereitung und/oder gegebenenfalls zur Durchführung eines Folgeverfahrens, insbesondere einer Vervielfältigungsreaktion für einen oder mehrere Analyten aus einer biologischen Probe sowie gegebenenfalls zur Durchführung einer Analyse eines oder mehrerer Analyten aus einer biologischen Probe, zumindest ein Modul zur Aufnahme und/oder Ausgabe zumindest eines Probengefäßes, zumindest ein Modul zum Transport eines oder mehrerer Reaktionsgefäße, zumindest ein Modul zur Probenaufbereitung, bei dem es sich um ein Lysemodul und/oder um ein Extraktionsmodul, das vorzugsweise eine magnetische Separation durchführt, handelt, zumindest ein Modul zur Vorbereitung eines Folgeverfahrens, insbesondere einer Vervielfältigungsreaktion und/oder eines Analyseverfahrens für einen oder mehrere Analyten, optional zumindest ein Modul zur Durchführung eines Folgeverfahrens, insbesondere einer Vervielfältigungsreaktion und/oder eines Analyseverfahrens für einen oder mehrere Analyten, und optional zumindest ein Modul zur Durchführung eines Nachweises eines oder mehrerer Analyten. Die Module sind auf mindestens zwei Einheiten aufgeteilt, wobei das Modul zur Probenaufbereitung in einer ersten Einheit und das Modul zur Vorbereitung eines Folgeverfahrens, insbesondere einer Vervielfältigungsreaktion und/oder eines Analyseverfahrens für einen oder mehrere Analyten in einer zweiten Einheit angeordnet sind.

Ein weiterer Aspekt der Erfindung betrifft eine Vorrichtung zur Probenverarbeitung, insbesondere zur Probenaufbereitung sowie zur Vorbereitung und/oder gegebenenfalls zur Durchführung eines Folgeverfahrens, insbesondere einer Vervielfältigungsreaktion für einen oder mehrere Analyten aus einer Probe sowie gegebenenfalls zur Durchführung einer Analyse eines oder mehrerer Analyten aus einer Probe, die zumindest ein Modul zur Aufnahme und/oder Ausgabe eines oder mehrerer Probengefäße, zumindest ein Modul zum Transport eines oder mehrerer Reaktionsgefäße, zumindest ein Modul zur Probenaufbereitung, bei dem es sich um ein Lysemodul und/oder um ein Extraktionsmodul, das vorzugsweise eine magnetische Separation durchführt, handelt, zumindest ein Modul zur Vorbereitung eines Folgeverfahrens, insbesondere einer Vervielfältigungsreaktion und/oder eines Analyseverfahrens für einen Analyten, optional zumindest ein Modul zur Durchführung eines Folgeverfahrens, insbesondere einer Vervielfältigungsreaktion für einen oder mehrere Analyten, und optional zumindest ein Modul zur Durchführung einer Analyse eines oder mehrerer Analyten umfasst. Die Module sind auf mindestens zwei Einheiten aufgeteilt und besitzen jeweils eine eigene Steuerung, wobei die mindestens zwei Einheiten durch einen ersten Datenbus verbunden sind.

Die erfindungsgemäße Vorrichtung bietet eine Reihe von Vorteilen, die sich vor allem aus der strukturellen Aufteilung in Einheiten und Module ergeben. Die Vorrichtung ist frei konfigurierbar und kann sowohl bei Bestellung als auch im Nachhinein flexibel konfiguriert werden. Insbesondere lassen sich verschiedene Bearbeitungstiefen abhängig von den Bedürfnissen eines Anwenders realisieren. So sind beispielsweise abhängig von der Konfiguration der Vorrichtung folgende komplett abgebildete Prozesstiefen realisierbar: Ausgehend von einer Probe in einem beliebigen Probengefäß bis zur Extraktion eines Analyten nach Lyse der Probe, alternativ bis zur Vorbereitung eines Folgeverfahrens wie einer Vervielfältigungsreaktion beispielsweise einer Polymerase-Kettenreaktion (PCR) oder eines Protein-Assays durch Reagenzienzugabe, alternativ bis zur Durchführung einer PCR, oder alternativ bis zum Abschluss der Analyse, d.h. bis zum fertigen Analyseergebnis (Primary tube to result).

Unter Probenaufbereitung werden insbesondere die Verfahren verstanden, die von der Aufgabe der Probe bis zum aufgereinigten Analyten fuhren. Dies umfasst beispielsweise Lyseverfahren, insbesondere chemische oder enzymatische Lyseverfahren, und Extraktionsverfahren, die vorzugsweise das Binden einer oder mehrerer Zielkomponenten an eine Matrix, optional das Waschen der Matrix zum Entfernen von unerwünschten Komponenten von der Matrix, das Abtrennen der Matrix mit den gebundenen Zielkomponenten und das Desorbieren/Lösen (Eluieren) der Zielverbindung von der Matrix einschließen.

Insbesondere wenn sich die Zielverbindungen wie Nukleinsäuren und Proteine in schwer aufschließbaren biologischen Proben befinden, kann die Probenaufbereitung auch vorgeschaltete mechanische Aufschlussverfahren umfassen, die aber nicht unbedingt in derselben Einheit ablaufen müssen wie die anderen genannten Verfahren zur Probenaufbereitung. Generell sind sämtliche aus dem Stand der Technik bekannten Verfahren zur Medienvorbereitung und Isolierung und/oder Aufkonzentrierung von biologischen Zielkomponenten als Probenaufbereitungsverfahren denkbar.

Bei der zu verarbeitenden biologischen Probe kann es sich um eine gefrorene oder um eine nicht gefrorene biologische Probe handeln, wobei als biologische Probe alle dem Fachmann bekannten biologischen Proben eingesetzt werden können. Bevorzugte biologische Proben sind ausgewählt aus der Gruppe umfassend Biomoleküle, beispielsweise natürliche, vorzugsweise isolierte lineare, verzweigte oder zirkuläre Nukleinsäuren wie RNA, insbesondere mRNA, siRNA, miRNA, snRNA, tRNA, hnRNA oder Ribozyme, DNA und dergleichen, synthetische oder modifizierte Nukleinsäuren, beispielsweise Oligonukleotide, insbesondere für die PCR verwendete Primer, Sonden oder Standards, mit Digoxigenin, Biotin oder Fluoreszensfarbstoffen markierte Nukleinsäuren oder sogenannte PNAs (*"peptide nucleic acids"*)*,* natürliche, vorzugsweise isolierte Proteine oder Oligopeptide, synthetische oder modifizierte Proteine oder Oligopeptide, beispielsweise mit Fluoreszenzmarkern oder Enzymen gekoppelte Antikörper, Körperflüssigkeiten wie Blut, Sperma, Cerebrospinalflüssigkeit, Speichel, Sputum oder Urin, Flüssigkeiten, welche beim Aufarbeiten von Blut erhalten werden, wie etwa Serum oder Plasma, Leukozyten-Fraktionen oder "buffy coat", Blutegelspeichel (Saliva), Fäkalien, Abstriche, Punktate, Schuppen, Haare, Hautfragmente, forensische Proben, Lebensmittel- oder Umweltproben, die freie oder gebundene Biomoleküle, insbesondere freie oder gebundene Nukleinsäuren enthalten, Stoffwechselprodukte, ganze Organismen, vorzugsweise ganze nicht lebende Organismen, Gewebe von Mehrzellern, vorzugsweise von Insekten und Säugetieren, insbesondere vom Menschen, beispielsweise in Form von Gewebeschnitten oder Organen, isolierte Zellen, beispielsweise in Form adhärenter oder suspendierter Zellkulturen, Organellen, beispielsweise Chloroplasten oder Mitochondrien, Vesikel, Zellkerne oder Chromosomen, Pflanzen, Pflanzenteile, Pflanzengewebe oder Pflanzenzellen, Bakterien, Viren, Viroide, Prionen, Hefen und Pilze.

Als eine nicht gefrorene biologische Probe wird vorzugsweise eine frisch hergestellte biologische Probe eingesetzt, beispielsweise eine frische Gewebeprobe oder frisch isolierte Blutzellen aus einem lebendigen oder toten Organismus oder, im Falle synthetischer Biomoleküle als biologische Probe, frisch synthetisierte Nukleinsäuren oder Proteine. Unter einer "frischen" biologischen Probe wird dabei erfindungsgemäß vorzugsweise eine Probe verstanden, die vor nicht mehr als 96 Stunden entnommen oder, im Falle eines synthetischen Biomoleküls, synthetisiert worden ist. Die Bezeichnung "frische" biologische Probe umfasst jedoch auch solche Proben, die innerhalb des vorstehend genannten Zeitraums entnommen worden sind, die jedoch zum Stabilisieren noch vorbehandelt worden sind, beispielsweise mit herkömmlichen Fixativen, wie etwa Formalin, mit Farbstoffen, wie etwa Eosin, mit Antikörpern und dergleichen. Die Herstellung frischer Zell- oder Gewebeproben kann dabei durch alle dem Fachmann zu diesem Zweck bekannten Präparationsverfahren erfolgen, im Falle einer Gewebeprobe beispielsweise mittels eines Skalpells, etwa bei einer Operation oder einer Autopsie, im Falle einer Blutzellprobe durch Zentrifugation von frisch entnommenem Blut und dergleichen.

Als eine gefrorene biologische Probe wird vorzugsweise eine biologische Probe eingesetzt, die, nachdem sie auf die zuvor beschriebene Art und Weise isoliert worden ist, auf Temperaturen von 0°C oder weniger, vorzugsweise auf Temperaturen von -20°C oder weniger und am meisten bevorzugt auf Temperaturen von -70°C oder weniger, etwa durch das in Kontakt bringen mit flüssigem Stickstoff, abgekühlt worden ist.

Die Probe kann dabei in jeglicher Form von aus dem Stand der Technik bekannten Gefäßen der vorliegenden Vorrichtung zugeführt werden. Normalerweise handelt es sich dabei um ein offenes Primärgefäß. Bei Blutproben erfolgt dies üblicherweise in einem geschlossenen Primärröhrchen. Es ist ebenfalls denkbar, der Vorrichtung eine Probe zuzuführen, die bereits vorbehandelt wurde. Bei der Vorbehandlung kann es sich dabei beispielsweise um ein Überführen der Probe, die sich in einem verschlossenen Primärgefäß befindet, in ein Prozessgefäß oder ein anderes Gefäß mit leichtem Zugang zur Probe handeln. Darüber kann auch vorab eine Behandlung der Probe stattgefunden haben, wie ein mechanischer Zellaufschluss, oder eine enzymatische und/oder chemische Lyse. In diesem Fall würde auf der Vorrichtung selber vorzugsweise kein Lyseschritt mehr stattfinden.

Bei den Analyten, die nach der Probenaufbereitung vorzugsweise isoliert vorliegen und einem Folgeverfahren unterzogen werden, handelt es sich vorzugsweise natürliche, modifizierte oder synthetische Nukleinsäuren oder natürliche, modifizierte oder synthetische Proteine oder Oligopeptide. Als Nukleinsäuren kommen alle dem Fachmann bekannten Nukleinsäuren in Betracht, insbesondere Ribonukleinsäuren (RNA), beispielsweise mRNA, siRNA, miRNA, snRNA, t-RNA, hnRNA oder Ribozyme, oder Desoxyribonukleinsäuren (DNA). Grundsätzlich kann es sich um jeden Typ von Polynukleotid handeln, der ein N-Glykosid oder C-Glykosid einer Purin- oder Pyrimidinbase darstellt. Die Nukleinsäure kann einzel-, doppel- oder mehrsträngig, linear, verzweigt oder zirkulär sein. Sie kann einem in einer Zelle vorkommenden Molekül entsprechen, wie etwa genomische DNA oder Boten-RNA (mRNA), oder *in vitro* erzeugt werden wie Komplementär-DNA (cDNA), Gegenstrang-RNA (aRNA), oder synthetische Nukleinsäuren. Die Nukleinsäure kann aus wenigen Untereinheiten, mindestens zwei Untereinheiten, bevorzugt acht oder mehr Untereinheiten bestehen, wie etwa Oligonukleotide, mehreren hundert Untereinheiten bis hin zu mehreren tausend Untereinheiten, wie etwa bestimmte Expressionsvektoren, oder bedeutend mehr Untereinheiten, wie genomische DNA. Bevorzugt enthält die Nukleinsäure die kodierende Information für ein Polypeptid in funktionellem Zusammenhang mit regulatorischen Sequenzen, die die Expression des Polypeptids in der Zelle erlauben, in die die Nukleinsäure eingebracht wird oder in der sie natürlich vorliegt. So ist die Nukleinsäure in einer bevorzugten Ausführungsform ein Expressionsvektor. In einer anderen Ausführungsform ist sie eine pDNA (Plasmid-DNA), eine siRNA, eine siRNA-Duplizes oder eine siRNA-hetero-Duplizes, wobei unter dem Begriff "siRNA" Ribonukleinsäuren mit einer Länge von etwa 22 Nukleotiden verstanden werden, die durch Spaltung einer doppelsträngigen RNA (dsRNA) durch das Enzym "Dicer" entstehen und in den Enzymkomplex "RISC" (RNA-induced silencing complex) eingebaut werden.

Unter Folgeverfahren werden vorzugsweise aus dem Stand der Technik bekannte Verfahren zur quantitativen und/oder qualitativen Analyse sowie Vervielfältigungsverfahren verstanden. Zu den Vervielfältigungsverfahren zählen dabei insbesondere die Polymerase-Kettenreaktion (PCR) und die Ligase-Kettenreaktion (LCR), sowie die WGA ("Whole Genome Amplification"), wobei zur PCR beispielsweise anchored, asymmetrische, Error prone, in situ, inverse, Long Range, Real Time und reverse Transkriptase zu nennen wären.

Als dem Fachmann bekannte und geeignet erscheinende Analyseverfahren können beispielsweise Verfahren ausgewählt aus der Gruppe umfassend die Lichtmikroskopie, die Elektronenmikroskopie, die konfokale Laserscanningmikroskopie, die Laser-Micro-Dissection, Scanningelektronenmikroskopie, das Western-Blotting, das Southern-Blotting, den Enzyme-linked Immonosorbent-Assay (ELISA), die Immunpräzipitation, die Affinitätschromatographie, die Mutationsanalyse, die Polyacrylamidgelelektrophorese (PAGE), insbesondere die zweidimensionale PAGE, die HPLC, die RFLP-Analyse (Restriction Fragment Length Polymorphism-Analyse), die SAGE-Analyse (Serial Analysis of Gen Expression), die FPLC-Analyse (Fast Protein Liquid Chromatography), die Massenspektrometrie, beispielsweise die MALDI-TOFF-Massenspektrometrie oder die SELDI-Massenspektrometrie, die Microarray-Analyse, die LiquiChip-Analyse, die Analyse der Aktivität von Enzymen, HLA-Typing, Sequenzierung, RNase-Protection-Analyse oder Primer-Extension-Analyse angeführt werden.

Auch Modifikationsreaktion wie Methylierungen zählen zu den Folgeverfahren im Sinne der Erfindung.

Gemäß einer bevorzugten Ausführungsform ist vorgesehen, dass die Reagenzien für eine Reihe definierter Prozesse in der Vorrichtung bevorratet sind, so dass beim Wechsel zwischen Prozessen für unterschiedliche Probenarten nur der gewünschte Prozess an einer Benutzerschnittstelle eingegeben werden muss, was die Bedienungsfreundlichkeit erhöht, den erforderlichen Personalaufwand mindert und damit hilft, Kosten zu senken.

Vorzugsweise ist vor dem Modul zur Probenaufbereitung ein weiteres Modul zur Probenaufbereitung vorgeschaltet, bei dem es sich nicht um ein Lyse- oder Extraktionsmodul handelt. Allgemein kann es sich bei dem vorgeschalteten Modul um alle aus dem Stand der Technik bekannten Verfahren zur Medienvorbereitung handeln. Vorzugsweise stellt das vorgeschaltete Modul ein Modul zur Durchführung von physikalischen Zellaufschlussverfahren oder zur Entnahme der Probe aus verschlossenen Primär-Probengefäßen dar, das zudem bevorzugt in einer weiteren Einheit bereitgestellt ist. Mit physikalischen Zellaufschlussverfahren sollen insbesondere nicht-mechanische Verfahren unter Ausnutzung des osmotischen Drucks, Temperaturerhöhung, Gefriertrocknung und/oder mechanische Verfahren z. B. unter Verwendung von Ultraschall, Mahlwerken oder Homogenisatoren verstanden werden.

Ein optionales Modul zur Durchführung einer Vervielfältigungsreaktion ist bevorzugt in einer weiteren Einheit bereitgestellt. Vorteilhafte Vervielfältigungsverfahren wurden bereits oben erwähnt.

Vorzugsweise befinden sich die Einheiten jeweils in eigenen Gehäusen, die im Wesentlichen baulich getrennt und besonders bevorzugt lösbar miteinander verbunden sind. Nach Verbindung der einzelnen Einheiten miteinander entsteht für einen Betrachter vorteilhafterweise der Eindruck, dass es sich um einziges Gerät handelt.

Vorzugsweise weist mindestens eine der Einheiten einen oder mehrere Bereiche für die Lagerung von unbenutzten und/oder benutzten Verbrauchsmaterialien auf, in dem diese in optional verschließbaren Behältern gelagert werden. In einem bevorzugten Ausführungsbeispiel sind die Bereiche für benutzte und unbenutzte Verbrauchsmaterialien getrennt ausgeführt.

Unter Verbrauchsmaterialien sollen alle Materialien verstanden werden, die nicht dauerhaft Bestandteil der Vorrichtung oder der einzelnen Einheiten bzw. Module sind, und die üblicherweise nach dem direkten Gebrauch oder nach ihrer Leerung, wenn es sich beispielsweise um Gefäße handelt, entsorgt werden. Beispiele für Verbrauchsmaterialien schließen Pipettenspitzen und andere z. B. für die Extraktion verwendete Einwegartikel (wie Magnetabschirmhülsen), sämtliche verwendeten Reagenzien (wie Puffer, Lysereagenzien, Waschlösungen und/oder Adsorptionsmaterialien), Prozessgefäße (die vorzugsweise Reaktionsgefäße und andere für den Prozessverlauf benötigte Gefäße wie Wasch-, Misch- und/oder Elutionsgefäße), Vorratsgefäße, Lagerungsbehältnisse (sowohl für unbenutzte Reagenzien und Zwischenprodukte der Verarbeitungsverfahren als auch für benutzte oder unbenutzte Einwegartikel und Abfallprodukte) ein.

Vorzugsweise besitzt mindestens eine der Einheiten ein Pipettiermodul für den Transfer einer biologischen Probe zwischen zwei Gefäßen, insbesondere einem Probengefäß und einem oder mehreren Prozessgefäßen.

Bevorzugt kommen während der Durchführung eines Prozesses innerhalb einer Einheit mehrere Prozessgefäße zum Einsatz. Dabei werden die Proben bzw. die Prozessmischung wiederholt von einem Prozessgefäß in ein weiteres umgefüllt, bevorzugt durch das Pipettiermodul. Alternativ ist auch die durchgehende Verwendung eines Reaktionsgefäßes während des Prozesses in einer Einheit möglich. In diesem Fall wird die Probe lediglich aus einem Eingangs-Probengefäß in ein Prozessgefäß transferiert und am Ende des Prozesses wiederum in ein Ausgangs-Probengefäß umgefüllt. Alternativ kann auch, statt eines Ausgangs-Probengefäßes, ein Prozessgefäß direkt an eine weitere Einheit übergeben werden oder als Ausgabe-Probengefäß dienen.

Das verwendete Reaktionsvolumen kann gegenüber dem Stand der Technik bzw. marktüblichen Geräten deutlich erhöht werden, was eine höhere Sensitivität der Vorrichtung ermöglicht. Beispielsweise werden aus der Probe 50 µl bis 10 ml, vorzugsweise 0,1 bis 5 ml, besonders bevorzugt 0,5 ml bis 3 ml in ein Reaktionsgefäß umgefüllt, in einem ganz besonders bevorzugten Ausführungsbeispiel 1 ml.

Nach der Extraktion kann die zur Elution verwendete Menge an Eluierungsmittel so gewählt werden, dass die gewünschte Aufnahmekonzentration im Eluat erhalten wird. Die Menge an Eluierungsmittel sollte natürlich groß genug gewählt werden, um zu große Ausbeuteverluste auf dem zur Extraktion verwendeten Trägermaterial zu vermeiden.

Es kann vorteilhaft sein, in der Probenaufbereitungseinheit oder dieser nachgeschaltet ein Normalisierungsmodul zu ergänzen. Dieses misst üblicherweise mittel Photometrie die Konzentration des Analyten im Eluat. Normalisierungsmodule als solche sind aus dem Stand der Technik bekannt. Nach der Messung der Analytkonzentration kann dann durch Verdünnen eine definierte Konzentration zur Durchführung ausgewählter Folgeverfahren hergestellt werden.

Vorteilhafterweise werden bei Zuschaltung eines Normalisierungsmoduls direkt beim Auffangen des Eluates UV-durchlässige Prozessgefäße verwendet, oder die Elutionslösung wird in solche Gefäße umgefüllt.

Generell kann ein solches Normalisierungsmodul in eine der vorhandenen Einheiten integriert werden, z. B. als Abschluss der Einheit zur Probenaufbereitung oder zu Beginn der Einheit zur Vorbereitung eines Folgeverfahrens. Zum Verdünnen selber könnten dann beispielsweise das Pipettiermodul der Einheit verwendet werden, in dem sich das Normalisierungsmodul befindet. Befindet sich das Normalisierungsmodul in der Einheit zur Probenaufbereitung, kann das Verdünnen auch in der nachgeschalteten Einheit zur Vorbereitung eines Folgeverfahrens stattfinden. Letzteres ist besonders bevorzugt, da die Einheit zur Vorbereitung von Folgeverfahren üblicherweise ein Pipettiermodul mit einer höheren Genauigkeit aufweist und trotz des Verdünnens üblicherweise sehr hohe Konzentrationen gewünscht werden.

Es ist ebenfalls möglich, das Normalisierungsmodul optional zusammen mit einem Pipettiermodul in einer separaten Einheit unterzubringen, die sich zwischen der Einheit zur Probenaufbereitung und der Einheit zur Vorbereitung eines Folgeverfahrens befinden sollte.

In einem bevorzugten Ausführungsbeispiel der Erfindung weist mindestens eine Einheit einen Mikroprozessor auf. Ein erster Datenbus, bevorzugt nach dem Ethernet-Standard, verbindet die Einheiten untereinander, wobei verschiedene Topologien möglich sind, vorzugsweise ein von einer Einheit ausgehender Stern, ein Ring oder die Verbindung über einen so genannten "Switch". Die Module innerhalb einer Einheit sind vorzugsweise über einen zweiten Datenbus verbunden, bevorzugt über einen CAN-Bus. Die Module innerhalb einer Einheit weisen bevorzugt eine einheitliche Schnittstelle zu dem zweiten Datenbus auf. In einer bevorzugten Ausführungsform weist mindestens eines der Module innerhalb einer Einheit einen Mikroprozessor, vorzugsweise einen Mikrocontroller auf. In einem weiteren bevorzugten Ausführungsbeispiel arbeitet mindestens ein Mikrocontroller mit einem Echtzeit-Betriebssystem.

Vorzugsweise ist ein Modul zur Aufnahme der Probengefäße mittels Adaptern zur Aufnahme einer Mehrzahl von unterschiedlichen Probengefäßen anpassbar, bei denen es sich vorzugsweise um handelsübliche Proben- bzw. Reaktionsgefäße wie ganz allgemein Test-Tubes (Röhrchen) verschiedener Größen, Multiwell-Probenplatten (z. B. 96-, 48-, 16-, 12- oder 8-Well-Platten), PCR-Tubes oder Blutentnahmeröhrchen handeln kann.

Neben der Aufnahme kann mindestens eine Einheit Probengefäße in verschiedenen Ausgangsformaten an eine weitere Einheit oder zur manuellen Weiterverarbeitung liefern. Während aus dem Stand der Technik bekannte Probenaufbereitungseinheiten und/oder Einheiten zur Vorbereitung bzw. Durchführung von Folgeverfahren auf Grund festgelegter Aufnahme- und Ausgabe-Formate, die bei Geräten verschiedener Anbieter meist variieren, üblicherweise nur in einer Kombination miteinander kompatibel sind, ist es vorliegend möglich, verschiedene Aufnahme- und Ausgabe-Formate verschiedener Anbieter auszuwählen. Somit können Einheiten der vorliegenden Vorrichtung, insbesondere die Einheit zur Probenaufbereitung und die Einheit zur Vorbereitung von Folgeverfahren, mit verschiedenen nachfolgenden und/oder vorgeschalteten Einheiten anderer Hersteller kombiniert werden und dennoch einen durchgehenden Prozessverlauf ermöglichen. Für PCR-Reaktionen als Folgeverfahren können insbesondere sämtliche bei herkömmlichen Cyclern zum Einsatz kommenden PCR-Tubes und PCR-Kapillaren als Ausgabe-Format dienen.

Die Transferierung der aufgearbeiteten und/oder verarbeiteten Proben in das gewünschte Ausgabeformat erfolgt vorzugsweise noch auf der Einheit, auf der auch die Probenaufbereitung, insbesondere die Lyse und/oder Extraktion, stattfindet. Es wäre auch denkbar die Transferierung auf der Einheit zur Vorbereitung des Folgeverfahrens durchzuführen.

Bevorzugt ist mindestens eine Einheit der Vorrichtung zur Verbindung mit einem externen Computer angepasst, so dass Parameter der Vorrichtung eingestellt und abgerufen werden können, bevorzugt Parameter zu Prozessabläufen, Typen von Probengefäßen und/oder Prozessgefäßen und Parameter zu Verbrauchsmaterialien sowie Logfiles und Statusberichte der Vorrichtung etc.

Vorzugsweise arbeitet ein Modul zur Probenaufbereitung als Extraktionsmodul, das nach einem Magnetseparationsverfahren arbeitet. Es ist vorzugsweise zur Befestigung einer Anordnung von Hülsen adaptiert, die zur räumlichen Trennung zwischen einer Probe in einem Reaktionsgefäß und einem zur Magnetseparation verwendeten Magneten dient, vorzugsweise einem Dauermagneten. Die Hülsen sind vorzugsweise in einer Matrix oder mehreren Matrizen zu einer Gesamtheit angeordnet.

Alternativ kann das Extraktionsmodul auch mit anderen aus dem Stand der Technik bekannten Verfahren zur Separation des Trägermaterials bzw. zur Magnetseparation arbeiten.

Vorzugsweise weist mindestens eine der Einheiten eine Benutzerschnittstelle auf. Diese kann bevorzugt in Form eines Touch-Screens realisiert sein und dient zur Eingabe von Anweisungen durch eine Bedienperson. Gleichzeitig werden auf dem Monitor Statusinformationen, Eingabeaufforderungen und eventuelle Fehlermeldungen der Vorrichtung angezeigt. Gemäß einer weiteren bevorzugten Ausführungsform ermöglicht ein Fernwartungsmodul die Durchführung von Fernwartung und Ferndiagnose, beispielsweise über das Internet, etwa durch einen Wartungsdienst oder den Hersteller.

Ein weiterer Aspekt der Erfindung betrifft eine Einheit zur Probenverarbeitung, die ein Modul zur Aufnahme und/oder Ausgabe eines oder mehrerer Probengefäße und ein oder mehrere identische oder voneinander verschiedene weitere Module aufweist, die aus einer Gruppe ausgewählt sind, die umfasst: (a) ein Modul zum Transport eines oder mehrerer Reaktionsgefäße innerhalb der Einheit, (b) ein Transfermodul für den Transport eines oder mehrerer Prozessgefäße zwischen zwei Einheiten, wobei das Transfermodul vorzugsweise einen Teleskoparm und/oder ein Förderband aufweist und vorzugsweise bidirektional arbeitet, (c) ein Modul zur Probenaufbereitung, bei dem es sich um ein Lysemodul und/oder ein Extraktionsmodul, welches vorzugsweise eine magnetische Separation durchführt, handelt, (d) ein Modul zur Vorbereitung eines Folgeverfahrens, insbesondere einer Vervielfältigungsreaktion und/oder eines Analyseverfahrens, bei dem es sich vorzugsweise um ein Pipettiermodul und/oder ein Kühlmodul handelt, (e) ein Modul zur Vervielfältigung und/oder zur Analyse eines oder mehrerer Analyten und (f) ein Modul zur Aufbewahrung der verarbeiteten Proben, insbesondere der aufbereiteten Proben, sowie (g) ein von (c) verschiedenes Modul zur Probenaufbereitung, bei dem es sich vorzugsweise um ein Modul zum physikalischen Zellaufschluss oder zur Entnahme der Probe aus verschlossenen Primär-Probengefäßen handelt. Dabei befinden sich die Module (c) und (d) nicht in einer Einheit. Die Einheit weist eine erste Steuerung auf und kommuniziert mit einer zweiten Einheit über einen Datenbus, während die Module innerhalb einer Einheit jeweils über einen weiteren Datenbus kommunizieren.

Jede solcher Einheiten befindet sich vorteilhafterweise in einem separaten, im Wesentlichen abgeschlossenen Gehäuse. Die Gehäuse können jedoch lösbar miteinander verbunden werden. Ebenso wie bei einer einzelnen der beschriebenen Einheiten, die vorzugsweise für sich abgeschlossen ist, vermittelt auch die Kombination mehrerer Einheiten dem Betrachter den Eindruck, dass es sich um ein einziges Gerät handelt.

Bevorzugt weist mindestens eine Einheit der Vorrichtung einen oder mehrere Bereiche zur Lagerung von benutzten und/oder unbenutzten Verbrauchsmaterialien auf, wobei der oder die Bereiche zur Lagerung der unbenutzten Verbrauchsmaterialien vorzugsweise räumlich von dem oder den Bereichen zur Lagerung der benutzten Verbrauchsmaterialien getrennt ist (sind).

Bevorzugt umfasst die Vorrichtung eine Steuerung, die spezifische Informationen zu Prozessen und/oder Prozessparametern enthält und die in Abhängigkeit vorab definierter und vor dem Start des Prozesses bestimmter Eigenschaften der Verbrauchsmaterialien und des gewählten Prozesses überprüft, ob der gewählte Prozess durchführbar ist, und falls erforderlich auf Diskrepanzen hinweist und/oder Möglichkeiten zur Lösung der Diskrepanzen anbietet.

Es ist weiter bevorzugt, dass mindestens eine der Einheiten der Vorrichtung eine Steuerung umfasst, die spezifische Informationen zu verschiedenen Arten von Verbrauchsmaterialien enthält, vorzugsweise zu deren benötigter Menge für einen geplanten Prozessablauf sowie zu der Form, den Abmessungen, dem Fassungsvermögen und dem Inhalt von Probengefäßen, Prozessgefäßen (wie Reaktions-, Wasch-, Elutions- und/oder Mischgefäßen) und/oder Vorratsgefäßen, und wobei diese Informationen nach Bestimmung einer oder mehrerer vorab definierter Eigenschaften der Verbrauchsmaterialien vor Beginn eines Prozessablaufs, vorzugsweise bei Beladung der Vorrichtung, durch eine Sensorik, vorzugsweise durch einen Lasersensor und/oder eine Digitalkamera und/oder einen Ultraschallsensor für den Prozessverlauf genutzt werden.

Dies ermöglicht es dem Benutzer, vor dem Start des Prozesses eine Überprüfung der gesamten Beladung des Gerätes vornehmen zu lassen, die beispielsweise aufzeigen kann, ob ausreichend Einwegartikel und Reagenzien für den gewählten Prozess zur Verfügung stehen und/oder ob die Einwegartikel beispielsweise im Hinblick auf ihr Volumen für das ausgewählte Protokoll geeignet sind. Die Steuerung zeigt vorteilhafterweise direkt eine mögliche bzw. empfohlene Aktion des Anwenders an, um die angezeigte(n) Diskrepanz(en) zu beheben. Wenn diese Aktion vom Anwender durchgeführt wurde, kann er eine erneute Prüfung vornehmen lassen, um anschließend, wenn diese positiv ausfiel, den Prozess zu starten.

Vorzugsweise kommen während eines Prozessdurchlaufs zur Verarbeitung einer Probe in der Einheit, die das Modul zur Probenaufbereitung umfasst, mindestens 2, vorzugsweise geometrisch identische, Prozessgefäße und vorzugsweise mindestens ein weiteres geometrisch von diesen verschiedenes Probengefäß zum Einsatz, wobei es sich bei dem Probengefäß weder um ein Prozess- noch um ein Vorratsgefäß handelt.

Dabei wird die Probe oder zumindest ein Teil derselben in ein Prozessgefäß, wie z. B. ein Reaktionsgefäß, überführt, in dem beispielsweise die Lyse als Verfahren zur Aufbereitung stattfinden soll. Wahlweise kann bei bereits lysierten Proben die auf das Gerät aufgegeben werden, die lysierte Probe auch direkt in das Gefäß zur Extraktion eingebracht werden. Die Elution der separierten Trägermatrix mit dem gebundenen Analyten findet dann vorzugsweise in einem neuen Prozessgefäß statt.

Um zu vermeiden, dass verschiedene Gefäße mit unterschiedlicher Geometrie auf dem Gerät bereitgehalten werden müssen, was generell durchaus möglich ist, ist es besonders vorteilhaft, dass die Prozessgefäße in der Einheit zur Probenaufbereitung, insbesondere die Gefäße, in denen die Lyse, die Extraktion und das Eluieren stattfinden, dieselbe geometrische Form aufweisen. Optional können vor dem Eluieren ein oder mehrere Waschschritte stattfinden, die ebenfalls bevorzugt in jeweils einem neuen Gefäß mit identischer geometrischer Form stattfinden.

Standardmäßig werden die Eluate in eine Multiwell-Platte, beispielsweise eine 96-Well-Platte, zur Ausgabe aus der Einheit zur Probenaufbereitung überführt. Optional kann das Überführen jedoch auch in ein anderes Format erfolgen, das für das gewünschte Folgeverfahren besonders geeignet ist.

Bevorzugt kommen in der Einheit, die das Modul zur Vorbereitung eines Folgeverfahrens, insbesondere einer Vervielfältigungsreaktion und/oder eines Analyseverfahrens umfasst, ein Prozessgefäß und ein oder mehrere weitere Prozess- und/oder Vorratsgefäße, die unabhängig voneinander untereinander und zum ersten Prozessgefäß geometrisch identisch oder verschieden sein können, zum Einsatz.

Je nach geplantem Folgeverfahren findet das gewählte Vorbereitungsverfahren vorzugsweise in dem Ausgabeprozessgefäß der vorherigen Einheit statt. Es ist jedoch auch möglich die Proben in andere Gefäße zu überführen. Insbesondere bei anschließender Durchführung einer PCR-Reaktion oder eines anderen Verfahrens, bei dem zunächst die Zugabe weiterer Reagenzien bzw. Mischungen erforderlich ist, wird die benötigte Reagenzienmischung, z. B. PCR-Mastermix, vorteilhafterweise bereits fertig gemischt bereitgestellt, beispielsweise in einem Vorratsgefäß, oder auf dem Gerät selber gemischt. Das Mischen der zuzugebenden Reaktionslösung kann dabei auch in einem Vorratsgefäß erfolgen, das das Reagenz für den gesamten Durchgang liefert. Es wäre auch denkbar, die benötigte gemischte Reagenzienlösung direkt in Prozessgefäßen bereitzustellen, die sowohl vorgefüllt als auch auf dem Gerät befüllt werden können. Das Eluat kann dann direkt in die mit Reagenzien gefüllten Prozessgefäße gegeben werden.

Vorzugsweise besitzt mindestens eine Einheit der Vorrichtung eine Transfereinrichtung für den Transport eines Probengefäßes zwischen zwei Einheiten, wobei dieses Transfermodul vorzugsweise einen Teleskoparm und/oder ein Förderband aufweist und vorzugsweise bidirektional arbeitet. Je nach technischer Ausgestaltung kann das Transfermodul sowohl den Transfer in die vorgeschaltete Einheit und die nachgeschaltete Einheit ermöglichen als auch nur in eine dieser Einheiten. Im letzteren Fall müsste die vor- bzw. nachgeschaltete Einheit, in die von der benachbarten Einheit kein Transfer möglich ist, ein eigenes Transfermodul aufweisen. Vorzugsweise weist zumindest die Einheit zur Probenaufbereitung ein Transfermodul auf.

Ein weiterer Aspekt der Erfindung betrifft ein Modul zur Verwendung in der oben beschriebenen Einheit zur biologischen Probenverarbeitung. Es umfasst eine Halterung für mindestens ein Gefäß, einen Mikrocontroller und eine Datenbus-Schnittstelle zur Daten-Kommunikation mit der Steuerung der Einheit.

Vorzugsweise besitzt mindestens ein Modul der Vorrichtung eine Transfereinrichtung für den Transport eines Probengefäßes zwischen zwei Einheiten, wobei dieses Transfermodul vorzugsweise einen Teleskoparm und/oder ein Förderband aufweist und vorzugsweise bidirektional arbeitet.

Weiterhin ist bevorzugt, dass ein Modul einen Festwertspeicher und/oder einen Arbeitsspeicher und/oder einen elektronischen Schaltkreis zur Kommunikationssteuerung umfasst. Vorzugsweise besitzt das Modul eine Schnittstelle zu einem CAN-Datenbus. Bevorzugt arbeitet der Mikrocontroller mit einem Echtzeit-Betriebssystem.

In einem alternativen Ausführungsbeispiel umfasst das Modul einen Mikroprozessor statt des Mikrocontrollers.

Da die oben beschriebenen Module vorzugsweise in einer oder mehreren der oben beschriebenen Einheiten enthalten sind und mindestens zwei dieser Einheiten wiederum in der Vorrichtung enthalten sind, gelten die Beschreibungen zu den jeweiligen Erfindungsgegenständen (Modul, Einheit, Vorrichtung) auch für die jeweils anderen Erfindungsgegenstände, so dies nicht explizit ausgeschlossen ist oder technisch keinen Sinn macht.

Im Folgenden werden die Einzelheiten der Erfindung anhand verschiedener Ausführungsbeispiele unter Bezugnahme auf die beigefügten Zeichnungen erläutert. Darin zeigt:
- Fig. 1: eine schematische Darstellung einer Vorrichtung gemäß einem Ausführungsbeispiel der vorliegenden Erfindung.
- Fig. 2: eine schematische Darstellung einer Einheit gemäß einem weiteren Ausführungsbeispiel der vorliegenden Erfindung.
- Fig. 3: eine schematische Darstellung einer weiteren Einheit gemäß einem weiteren Ausführungsbeispiel der vorliegenden Erfindung.
- Fig. 4: eine schematische Darstellung einer Vorrichtung gemäß einem weiteren Ausführungsbeispiel der vorliegenden Erfindung.
- Fig. 5: eine schematische Darstellung einer Vorrichtung gemäß einem weiteren Ausführungsbeispiel der vorliegenden Erfindung.
- Fig. 6: eine schematische Darstellung einer Einheit zur Zelldisruption gemäß einem weiteren Ausführungsbeispiel der vorliegenden Erfindung.
- Fig. 7: eine schematische Darstellung einer Einheit zur Vervielfältigung eines Analyten gemäß einem weiteren Ausführungsbeispiel der vorliegenden Erfindung.
- Fig. 8: eine schematische Darstellung einer Detektionseinheit gemäß einem weiteren Ausführungsbeispiel der vorliegenden Erfindung.
- Fig. 9: eine schematische Darstellung des Blockschaltbilds einer Vorrichtung gemäß einem weiteren Ausführungsbeispiel der vorliegenden Erfindung.
- Fig. 10: eine schematische Darstellung des Blockschaltbilds eines Steuerungsmoduls gemäß einem weiteren Ausführungsbeispiel der vorliegenden Erfindung.
- Fig. 11: eine schematische Darstellung des Blockschaltbilds der Mikrocontroller-Steuerung eines Moduls gemäß einem weiteren Ausführungsbeispiel der vorliegenden Erfindung.

Bei der folgenden Beschreibung verschiedener Ausführungsbeispiele der vorliegenden Erfindung sind funktional gleiche Merkmale der verschiedenen Ausführungsbeispiele mit denselben Bezugszeichen versehen.

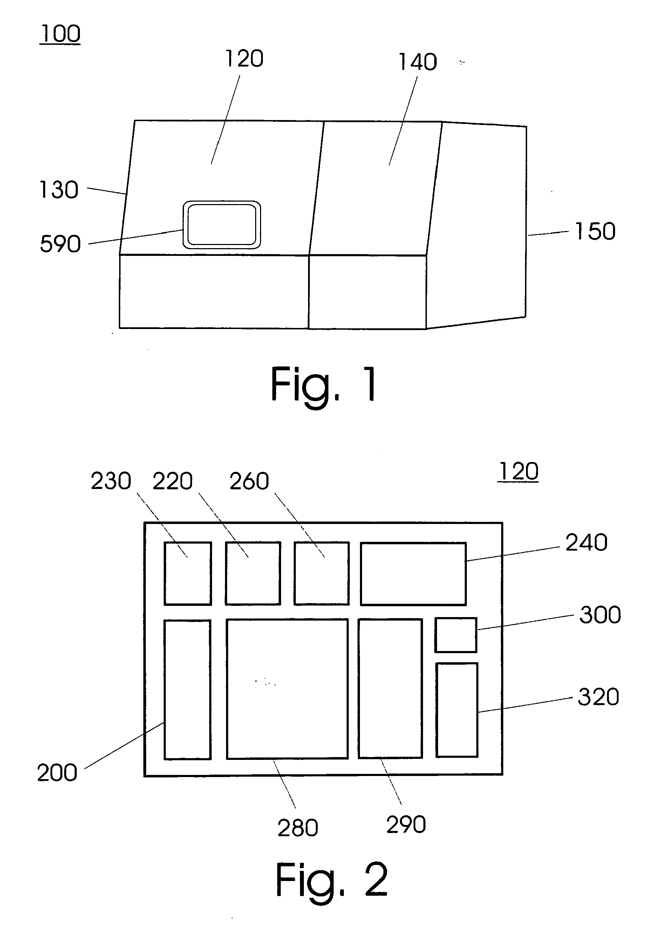

Fig. 1 zeigt eine typische Ausführungsform der Erfindung. Eine Vorrichtung (100) dient zum Isolieren und Aufkonzentrieren eines Analyten aus einer biologischen Probe und zum Vorbereiten eines Folgeverfahrens, wie beispielsweise einer Vervielfältigungsreaktion, Analyse und/oder einer Modifikation. Sie umfasst mehrere voneinander baulich getrennte Einheiten (120, 140), die sich in zwei Gehäusen (130, 150) befinden.

Fig. 2 zeigt eine schematische Darstellung einer Anzahl von Modulen, die in einer ersten Einheit (120) zusammengefasst sind. Diese umfasst ein Modul zur Aufnahme eines Probengefäßes (200); ein Modul (220) zur Lyse des Probenmaterials; ein Extraktions-Modul (240), das, nach Zugabe einer festen Adsorptionsmatrix in einem vorgeschalteten Schritt, das Abtrennen der gewünschten, an die Adsorptionsmatrix gebundenen Probenbestandteile von nichtgebundenen Probenbestandteilen, das Eluieren des Analyten von der Adsorptionsmatrix und das Abtrennen des Eluats von der Adsorptionsmatrix ermöglicht; ein Pipettiermodul (260) zum Aspirieren und Dispensieren von Reagenzien, Proben bzw. Prozessgemischen, wie der Lysemischung, der Waschlösung, des Eluates, und zum Umfüllen von Proben; mindestens einen Bereich (290) zur Lagerung von benutzten und räumlich getrennt davon einen Bereich (280) zur Lagerung von unbenutzten Reagenzien bzw. Prozessmischungen, Pipettenspitzen, Prozessgefäßen sowie Anordnungen von Hülsen, die in entsprechend geeigneten Behältern vorliegen können, wobei die Lagerungsbereiche sich auch außerhalb des Gehäuses, aber in irgendeiner Form damit verbunden befinden können; ein Greifer-Modul (230) zum Transport von Proben- und/oder Prozessgefäßen innerhalb der Einheit sowie Anordnungen von Hülsen innerhalb der Vorrichtung (100); und ein Transfermodul (300) zur Überführung eines den Analyten enthaltenden Probengefäßes an eine zweite Einheit (140) der Vorrichtung (100) oder ein Gerät eines Drittherstellers und optional wieder zurück.

Die Bedienperson stellt eine oder mehrere Proben in einem oder mehreren Probengefäßen durch eine Eingangsklappe oder Schublade in das Modul zur Probenaufnahme (200) der ersten Einheit (120) der Vorrichtung (100). Typischerweise sind dabei eine Mehrzahl von biologischen Proben in mehreren Vertiefungen (Wells) einer Probenplatte angeordnet, es sind allerdings erfindungsgemäß auch andere aus dem Stand der Technik bekannte Probengefäße und -halter vorgesehen, wie etwa ganz allgemein Test-Tubes (Röhrchen) verschiedener Größen, Multiwell-Probenplatten (z. B. 96-, 48-, 16-, 12- oder 8-Well-Platten), PCR-Tubes oder Blutentnahmeröhrchen ggf. mit entsprechenden Haltern. Alternativ kann das Probengut auch durch ein Transfermodul (300) einer vorgeschalteten weiteren Einheit (110), die etwa mit einem Modul (205) zur Disruption von Zellmaterial versehen ist, in das Modul zur Probenaufnahme (200) transferiert werden. Der Bediener gibt auf einem Touch-Screen der Bedienoberfläche (590) einen gewünschten Prozess ein. Auf dem Monitor lassen sich die Fortschritte des Prozesses und eventuelle Rück- oder Fehlermeldungen beobachten.

Gemäß einem Ausführungsbeispiel der Erfindung umfasst die Vorrichtung mindestens eine Steuerung (500), die spezifische Informationen zu verschiedenen Arten von Verbrauchsmaterialien aufweist, vorzugsweise zu deren benötigter Menge für einen geplanten Prozessablauf sowie zu der Form, den Abmessungen, dem Fassungsvermögen und dem Inhalt von Proben-, Prozess- und/oder Vorratsgefäßen. Die Steuerung ermittelt vor Beginn eines Prozessablaufs, vorzugsweise bei oder nach Beladung der Vorrichtung (100) durch einen Benutzer, mittels einer Sensorik Informationen zu einer oder mehreren vorab definierten Eigenschaften der Verbrauchsmaterialien. Die Sensoren sind dabei vorzugsweise als Lasersensor und/oder Digitalkamera und/oder Ultraschallsensor ausgeführt. Die von der Steuerung ermittelten Informationen werden für den weiteren Prozessverlauf genutzt.

Nach dem Start eines Durchlaufs werden eine Anzahl der Proben teilweise oder komplett mittels des Pipettiermoduls (260) in ein Prozessgefäß umgefüllt, das Vertiefungen (Wells) für Proben besitzt. Beispielsweise werden von den Proben jeweils 50 µl - 10 ml, vorzugsweise 0,1 bis 5 ml, besonders bevorzugt 0,5 ml bis 3 ml in ein Reaktionsgefäß umgefüllt, in einem ganz besonders bevorzugten Ausführungsbeispiel 1 ml. Das Reaktionsgefäß wurde vorher von dem Greifer des Greifermoduls (230) aus einem Vorratsbehälter im Lagerbereich (280) der Einheit (120) aufgenommen. Das Reaktionsgefäß wird von dem Greifermodul (230) in den Bereich des Lyse-Moduls (220) transportiert und dort abgesetzt. Mittels des Pipettiermoduls (260) werden dann die zur Lyse erforderlichen Reagenzien aus einem oder mehreren Vorratsbehältern im Lagerungsbereich (280) entnommen.

Sämtliche Verbrauchsmaterialien, die für die Prozesse erforderlich sind, können von der Bedienperson direkt nach dem Entfernen der Umverpackung im Lagerbereich (280) der Vorrichtung (100) deponiert werden. Während eines laufenden Verarbeitungsprozesses können weitere Probenträger in der Vorrichtung (100) deponiert werden, ohne Einfluss auf den laufenden Prozess.

Das erste zugegebene Reagenz dient beispielsweise zum Aufschluss der Zellen in der Probe, um für weitere Prozessschritte DNA-, RNA-, Proteine oder weitere Zellbestandteile zugänglich zu machen. Anschließend werden Partikel zugegeben, die eine Oberfläche mit einer adsorbierenden Substanz aufweisen, vorzugsweise in Form einer Suspension mit magnetischen oder magnetisierbaren Mikropartikeln oder - perlen, die an ihrer Oberfläche mit einer adsorbierenden Substanz versehen sind.

Der nach der Lyse in der Lösung befindliche Analyt, z.B. DNA, RNA und/oder ein Protein, bindet nun an die Oberfläche mit der adsorbierenden Substanz und damit an die Partikel. Danach erfolgt eine Magnetseparation im Extraktionsmodul (240). Dabei wird die Lösung mit den Mikropartikeln von den restlichen Bestandteilen der ursprünglichen Probe befreit, gefolgt von einem oder mehreren optionalen Waschschritten, um schließlich Partikel zu erhalten, an die der aufgereinigte Analyt gebunden ist.. Dazu wird in das Reaktionsgefäß mit den Mikropartikeln und der zu reinigenden Lösung von oben eine Anordnung von Kunststoffhülsen eingeführt, wobei die Hülsen einen kleineren Durchmesser als der minimale Durchmesser der Öffnungen der Reaktionsgefäße besitzen. Die Hülsen sind innen hohl, dünnwandig und an ihrem oben liegenden, von den Reaktionsgefäßen entfernten Enden offen. Sie sind typischerweise in einer Matrixanordnung auf einer gemeinsamen Trägerplatte angeordnet. Dabei sind verschiedene Ausführungsformen möglich, etwa mit 8, 16, 24 und 48 Hülsen. Vorzugsweise werden mehrere, vorzugsweise drei, zwei-mal-vier-Anordnungen gleichzeitig verwendet. Die Hülsenanordnungen werden innerhalb der Einheit (120) mittels aus dem Stand der Technik bekannter Sensorik erkannt und von einer Steuerung (500) verwaltet.

Die Hülsen tauchen in ihrer unteren Endposition in die Reaktionsgefäße ein. Von oben wird nun mittels des Extraktionsmoduls (240) vorzugsweise in jede der Hülsen der Anordnung ein dünner Stab eingeführt, der vorzugsweise als durchgehender Stabmagnet ausgestaltet ist. Typischerweise handelt es sich um einen Dauermagneten. Bei einer kostengünstigeren Variante ist nur das untere Ende des Stabes, das in die Flüssigkeit eintaucht, als Magnet ausgebildet. Ein Teil des Magnets befindet sich nun unterhalb des Pegels der Probenflüssigkeit in dem Prozessgefäß, mit der Folge, dass die magnetischen Partikel an die Außenseite der Kunststoffhülse gezogen werden und an dieser haften. Eine Vorrichtung, die nachfolgend auch zur Schwingungserzeugung verwendet wird, entfernt dann die Hülsen synchron mit den darin befindlichen Magnetstäben in senkrechter Richtung nach oben, wodurch die Partikel mit dem adsorbierten Analyten ebenfalls aus der Lösung entfernt werden. Danach werden die Stäbe in ein zweites Prozessgefäß (Waschgefäß) getaucht. Dieses wurde von einem Förderband an die Stelle des ersten Reaktionsgefäßes transportiert und ist mit einem Reagenz gefüllt, das zum Spülen der Adsorptionsmatrix zum Entfernen der unerwünschten Lösungsbestandteile geeignet ist. Um eine möglichst gründliche Spülung der Partikel durch das Reagenz zu erzielen, werden die Magnetstäbe anschließend aus den Hülsen nach oben herausgefahren. Die an den Hülsen haftenden Partikel unterliegen dann nicht mehr der Anziehung der Magnete, lösen sich daher von der Außenseite der Hülsen und sammeln sich durch die Schwerkraft in dem unterhalb der Hülse befindlichen Waschgefäß. In alternativen Ausführungsbeispielen der Erfindung sind auch andere Verfahren zur magnetischen Separation möglich, die aus dem Stand der Technik bekannt sind, etwa der Einsatz magnetisierbarer Partikel statt magnetischer Partikel und abschaltbare Elektromagnete anstelle von Dauermagneten, sowie andere Extraktionsverfahren.

Vorzugsweise wird die oben erwähnte Vorrichtung zur Schwingungserzeugung im Extraktionsmodul (240) verwendet, um die Hülsen im eingetauchten Zustand in Vibration zu versetzen. Diese Vibration überträgt sich auf die Probenflüssigkeit mit den Partikeln und bewirkt, dass die Partikel in der Wasch- bzw. Elutionslösung resuspendiert werden. Nach einer definierten Zeitdauer werden die Magnetstäbe wieder in die Hülsen eingefahren. Die Partikel werden erneut magnetisch an die Außenseite der Hülsen gezogen und mit diesen aus den Reaktionsgefäßen nach oben herausgefahren. Anschließend wird wie im ersten Durchgang ein weiteres Reaktionsgefäß antransportiert, das mit frischem Reagenz gefüllt ist. Diese Schritte des Spülens der Partikel werden mehrfach wiederholt, abhängig von den voreingestellten Parametern des spezifischen Prozesses. Alternativ ist es möglich, die mehrfachen Wasch- bzw. Spülvorgänge nicht auszuführen, sondern direkt von dem vorhergehenden Schritt des Aufschlusses des Probenmaterials mit einem weiteren Schritt fortzufahren, vorzugsweise dem unten beschriebenen Eluieren.

Um den adsorbierten Analyten von den Partikeln zu trennen, werden in einem weiteren Schritt die Hülsen mit den anhaftenden Partikeln in ein weiteres Prozessgefäß eingefahren. Dieses ist mit einem Reagenz gefüllt, das zur Abtrennung des Analyten von den Partikeln geeignet ist (Elution). Nach Lösen der Partikel von den Stäben und einer Vibrationsdurchmischung wie in den vorigen Schritten liegt nach diesem Schritt eine Lösung des Analyten in dem Elutions-Reagenz vor. Die Partikel werden nun ein letztes Mal durch das Einfahren der Magnetstäbe an die Hülse gebunden und nach oben aus der Lösung entfernt. Sie werden in einem Behälter im Entsorgungsbereich (290) der Einheit (120) entsorgt. Vorher wurde das vorliegende Eluat mittels des Pipettiermoduls aus dem Prozessgefäß entnommen und in ein Prozessgefäß mit gewünschtem Ausgabeformat transferiert, das sich in einem Ausgangs-Lagerbereich (320) der Einheit (120) befindet. Von dort kann das Ausgabe-Prozessgefäß mit den extrahierten Proben von einer Bedienperson für die weitere Bearbeitung herausgenommen werden, oder, vorzugsweise, an eine zweite erfindungsgemäße Einheit (140) zur Weiterverarbeitung weitergeleitet werden. Zu diesem Zweck ist ein Transfermodul (300) vorgesehen, das das Ausgabe-Prozessgefäß mittels einer aus dem Stand der Technik bekannten Transportvorrichtung, vorzugsweise eines bidirektionalen Teleskoparms oder eines bidirektionalen Transportbands, durch eine Öffnung vorzugsweise in der Seitenwand des Gehäuses (130) an ein Probenaufnahmemodul (200) der zweiten Einheit (140) weiterleitet.

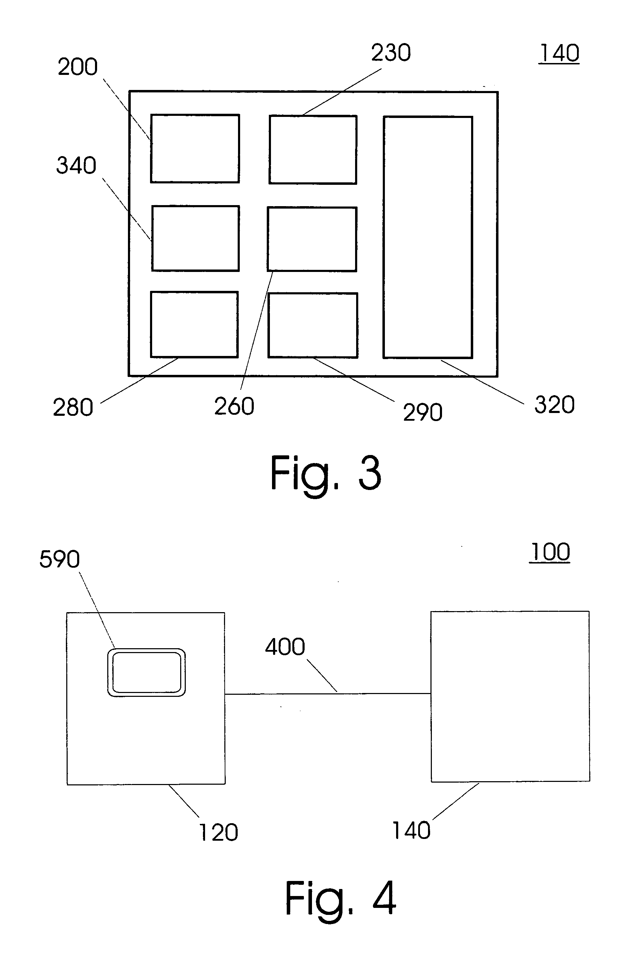

Fig. 3 zeigt eine schematische Darstellung einer zweiten Einheit (140) innerhalb der Vorrichtung (100) gemäß einem weiteren Ausführungsbeispiel der Erfindung. Die Einheit umfasst ein Modul zur Aufnahme von Prozessgefäßen (200), einen optional gekühlten Lagerbereich (340) für Reagenzien, einen Lagerbereich (280) zur Lagerung von Verbrauchsmaterialien wie Pipettenspitzen bzw. Prozessgefäßen, die sich vorzugsweise in entsprechenden Sammelbehältnissen befinden, sowie einen Entsorgungsbereich (290) für benutzte Verbrauchsmaterialien.

Ein Pipettier-Modul (260) dient zur Zugabe von Reagenzien zu der Probenlösung mit dem isolierten Analyten. Dadurch wird der Analyt zum Beispiel für die Durchführung einer Vervielfältigungsreaktion wie einer Polymerase-Kettenreaktion (PCR) vorbereitet oder ein Assay für die Analyse eines Proteins durchgeführt. Es ist auch möglich, mit verschiedenen Proben eines Laufs unterschiedlichen Folgeverfahren durchzuführen, wenn dies keine unterschiedlichen technischen Voraussetzungen erfordert. Die Information, welche Reagenzien für eine spezifische Probe anzuwenden sind, erhält die zweite Einheit (140) über die Datenbus-Verbindung (400) vom Steuerungsmodul der ersten Einheit (120). Ein Ausgangslagerbereich (320) dient zur Zwischenlagerung des den Analyten enthaltenden Ausgabeprozessgefäßes. Aus diesem Bereich kann das Probengefäß durch eine Bedienperson zur weiteren Bearbeitung entnommen werden. In einem typischen Ausführungsbeispiel dient ein optionales Transfermodul (300) zum Weiterleiten des Probengefäßes an eine weitere Einheit (160), in der ein Folgeverfahren, typischerweise eine Vervielfältigungsreaktion wie eine PCR oder eine Analyse, durchgeführt wird. Die zur Zugabe der Reagenzien zu den Proben verwendeten Pipetten werden nach einmaligem Gebrauch zur Vermeidung von Kreuzkontaminationen in einem oder mehreren Abfallbehältern innerhalb eines Lagerbereichs (290) der Einheit (140) entsorgt. Möglich wäre es auch, den Lagerbereich oder zumindest Teile davon außerhalb der Einheit bzw. der Vorrichtung vorzusehen, wobei der Lagerbereich lösbar mit der Einheit bzw. der Vorrichtung verbunden sein sollte.

Fig. 4 zeigt ein weiteres Ausführungsbeispiel der Erfindung, nach dem Einheiten (110, 120, 140, 160, 180) der Vorrichtung (100) über einen ersten Datenbus (400) verbunden sind. Dies ist in Fig. 4 schematisch für eine Vorrichtung mit zwei Einheiten (120, 140) dargestellt. Dabei sind verschiedene Topologien möglich, vorzugsweise ein von einer Einheit ausgehender Stern, ein Ring oder die Verbindung über einen gemeinsamen Switch. In einem Ausführungsbeispiel der Erfindung ist dieser Datenbus nach dem Ethernet-Standard realisiert.

Gemäß einem weiteren Ausführungsbeispiel wird die aufbereitete Probe aus der zweiten Einheit (140) durch ein Transfermodul (300) an eine weitere Einheit (160) übergeben. Diese umfasst ein PCR-Modul (170), optional auch ein Detektionsmodul (360) zur Detektion des Analyten. Das Detektionsmodul kann auch in einer weiteren Einheit (180) bereitgestellt werden.

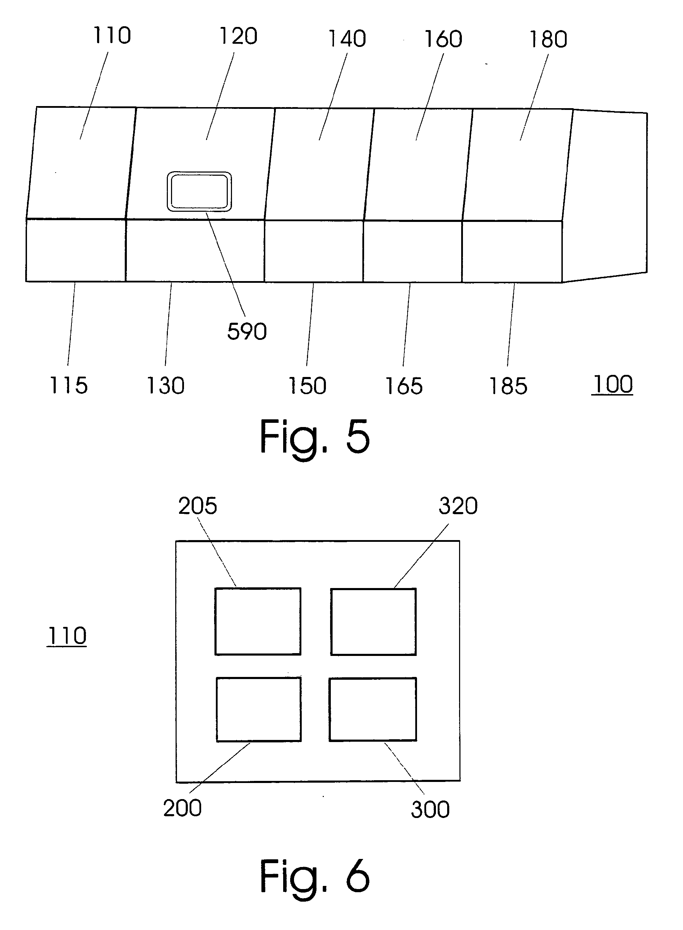

Fig. 5 zeigt ein Ausführungsbeispiel der Erfindung, bei dem die Vorrichtung (100) aus fünf Einheiten (110, 120, 140, 160, 180) aufgebaut ist, die jeweils ein eigenes Gehäuse (115, 130, 150, 165, 185) aufweisen, aber lösbar miteinander verbunden sein sollten. Nach außen entsteht vorzugsweise der Eindruck, dass es sich um ein einziges Gerät handelt.

Fig. 6 zeigt den schematischen Aufbau einer Einheit (110) zur physikalischen Zelldisruption gemäß einem Ausführungsbeispiel der Erfindung. Die Einheit umfasst mindestens ein Modul zur Probenaufnahme (200), ein Modul zur Zelldisruption (205), ein Transfermodul (300), das jedoch nur optional ist, sowie einen Ausgangs-Lagerbereich (320).

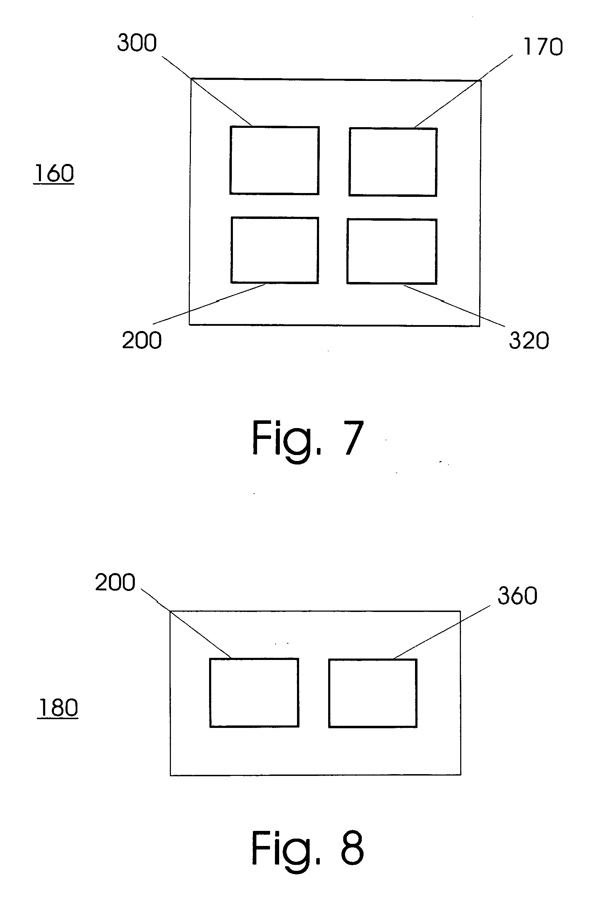

Fig. 7 zeigt den schematischen Aufbau einer Einheit (160) zur Durchführung einer Polymerase-Kettenreaktion gemäß einem Ausführungsbeispiel der Erfindung. Die Einheit umfasst mindestens ein Modul zur Probenaufnahme (200), ein PCR-Modul (170), ein Transfermodul (300) sowie einen Ausgangs-Lagerbereich (320).

Fig. 8 zeigt den schematischen Aufbau einer Einheit (180) zur Durchführung einer Detektion eines oder mehrerer Analyten gemäß einem Ausführungsbeispiel der Erfindung. Die Einheit umfasst mindestens ein Modul zur Probenaufnahme (200) sowie ein Detektions-Modul (360).

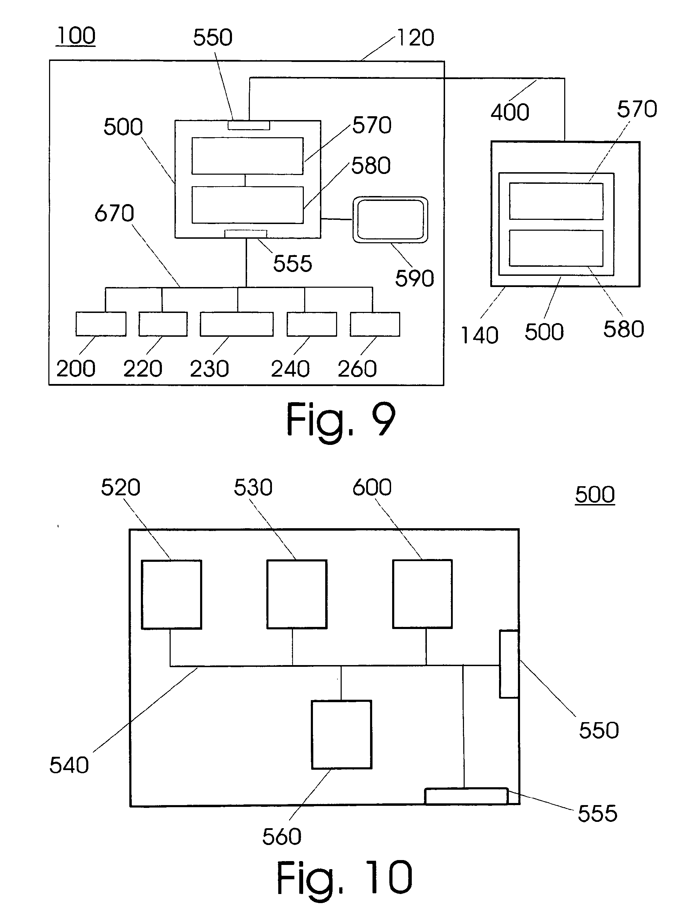

Fig. 9 zeigt eine schematische Darstellung einer Vorrichtung (100) gemäß einem Ausführungsbeispiel der Erfindung, wobei der Aufbau der ersten Einheit (120) mit ihren Modulen, in Verbindung mit einer schematisch gezeigten zweiten Einheit (140) gezeigt wird. Das Steuerungsmodul (500) ist die zentrale Instanz zur Steuerung der Vorgänge in einer Einheit (110, 120, 140, 160, 180). Es weist ein Betriebssystem (570) und mindestens eine Software-Applikation (580) auf, die zum Steuern und Regeln der Funktionen der Einzelmodule und deren Zusammenwirken dient. Über die externe Schnittstelle (550) kann das Steuerungsmodul über einen ersten Datenbus mit dem Steuerungsmodul einer zweiten Einheit (140) kommunizieren. Über eine zweite Schnittstelle (555) ist das Steuerungsmodul (500) über einen zweiten Datenbus (670) mit weiteren Modulen (200, 220, 230, 240, 260) innerhalb der Einheit (120) verbunden.

Fig. 10 zeigt das Blockschaltbild eines Steuerungsmoduls (500) einer Einheit gemäß einem Ausführungsbeispiel der Erfindung. Das Steuerungsmodul (500) ist typischerweise in Form eines Computers mit einem Mikroprozessor (520), einem Arbeitsspeicher (530), einem internen Bus (540), einer externen Schnittstelle (550) zu einem ersten Datenbus (400), und einem Steuerchipsatz (560) realisiert. Über eine zweite Schnittstelle (555) ist das Steuerungsmodul (500) über einen zweiten Datenbus (670) mit weiteren Modulen innerhalb der Einheit verbunden.

Wenn mehr als eine Einheit (110, 120, 140, 160, 180) der Vorrichtung (100) ein Steuerungsmodul (500) aufweisen, übernimmt eine der Einheiten die dominierende Rolle in einem Master/Slave-Betrieb. Die Eingabe von Befehlen einer Bedienperson an die Vorrichtung (100) wird über eine Bedienoberfläche (590) vorgenommen, die typischerweise in Form eines Touch-Screen an einer Frontseite des Gehäuses (130) der ersten Einheit (120) angebracht ist. Zu den Steuerbefehlen gehört etwa die Initialisierung der Vorrichtung (100), der Start eines Arbeitsdurchlaufs und die Eingabe, auf welche Analyten und ggf. mit welchem Verfahren die Proben untersucht werden sollen.

Sämtliche Prozessschritte werden von dem Steuerungsmodul (500) automatisch überprüft und verifiziert. Insbesondere wird nach dem Einstellen von Verbrauchsmaterialien überprüft, ob diese korrekt eingestellt wurden bzw. werden andere spezifische Information zu verschiedenen Arten von Verbrauchsmaterialien, die vorab definiert wurden, wie vorzugsweise deren benötigte Menge für einen geplanten Prozessablauf sowie zu der Form, den Abmessungen, dem Fassungsvermögen und dem Inhalt von Proben-, Prozess- und/oder Vorratsgefäßen, ermittelt, die zur Prüfung der Prozesserfordernisse dienen und Diskrepanzen zum ausgewählten Protokoll aufzeigen sollen. Ebenso werden nach dem automatischen oder manuellen Eingang eines Probengefäßes in den Eingangsbereich mittels Sensoren die Parameter des Probengefäßes festgestellt.

Das Steuerungsmodul sendet Steuersignale an die einzelnen Funktionsmodule und empfängt Informationen zu Status und Parametern der Module. Das Steuerungsmodul (500) initiiert in Einzelfällen auch direkte Kommunikation zwischen einzelnen Modulen, etwa bei Vorgängen wie dem unbefugten Öffnen des Gehäuse (130, 150) durch eine Person, bei dem eine schnelle Reaktion erforderlich ist. Der Datenbus (670) ist typischerweise ein in der Automatisierungstechnik standardmäßig verwendeter Datenbus, in einem bevorzugten Ausführungsbeispiel der Erfindung ein CAN (Controller Area Network). Die Module besitzen eine einheitliche Schnittstelle (680) zu dem Datenbus (670).

In einem Ausführungsbeispiel besitzt die Steuerung eine Speicherkarte (600) als Speichermodul für das Betriebssystem (570), die Applikationen (580) und gerätespezifische Daten wie Informationen über verschiedene Typen von Verbrauchsgütern. In einem weiteren Ausführungsbeispiel ist das Steuerungsmodul zusätzlich mit einer Festplatte ausgerüstet.

Gemäß einem weiteren Ausführungsbeispiel der Erfindung besitzt das Steuerungsmodul zusätzlich ein Modul zur Fernwartung, das Statusinformationen über das Internet zugänglich macht. Dadurch wird im Fehler- oder Wartungsfall auch eine Fernwartung und -diagnose möglich. In diesem Fall kann ein Techniker bereits das passende Austauschmodul zum Gerätestandort mitnehmen, was Servicekosten und Ausfallzeit zusätzlich reduziert.

Sämtliche Module sind erfindungsgemäß baulich voneinander getrennt und im Wesentlichen ohne die Demontage eines anderen Moduls aus einer Einheit (110, 120, 140, 160, 180) entnehmbar. Diese modulare Bauweise bietet mehrere Vorteile. Im Falle einer Betriebsstörung kann ein Servicetechniker schnell das fehlerhafte Modul identifizieren. Durch den Austausch gegen ein überholtes oder neues Ersatzmodul kann die Fehlerquelle mit minimalem Zeitaufwand und Ausfallzeit beseitigt werden.

Ein wesentlicher Vorteil der Modulbauweise ist die Aufrüstbarkeit des Geräts mit weiteren oder anderen Modulen. Ein Beispiel ist ein Eingangs-Modul, das zusätzlich die automatische Erkennung des Formats des Eingangs-Probengefäßes, vorzugsweise einer Multiwell-Probenplatte, über Barcodeabtastung oder Dimensionserkennung mittels Laserabtastung ermöglicht. Gleichzeitig kann das Gerät schnell und kostengünstig mit bisher nicht vorhandenen Funktionsmodulen in einer oder mehreren bereits vorhandenen Einheiten oder in Form einer kompletten, neuen Einheit nachgerüstet werden. So lässt sich die optionale weitere Einheit (160), in der z.B. eine PCR durchgeführt wird, nachträglich mit einem Detektionsmodul (360) versehen, um die Prozesskette von Probeneingabe bis Ausgabe des Analyseergebnisses vollständig in der Vorrichtung (100) abzubilden.

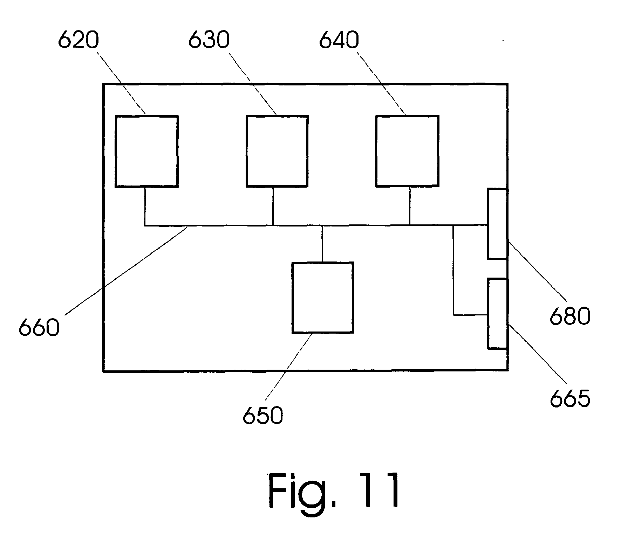

Nach einem weiteren Ausführungsbeispiel der Erfindung weisen einzelne Module jeweils einen Mikrocontroller (620) auf, der die Abläufe innerhalb des Moduls und die Kommunikation mit dem zentralen Steuerungsmodul (500) und/oder anderen Modulen steuert. Fig. 11 zeigt ein Blockschaltbild des Mikrocontrollers gemäß einem Ausführungsbeispiel der Erfindung. Der Mikrocontroller kann als Ein-Chip-Gerät realisiert sein oder mit weiteren Bauteilen wie Arbeitsspeicher (630), Festwertspeicher (640) und Input/Output-Chips (650) kombiniert sein, mit denen er über einen internen Bus (660) kommuniziert. Über eine Input/Output-Schnittstelle (665) kommuniziert der Mikrocontroller mit weiteren Bestandteilen des Moduls, wie etwa Sensoren und Aktoren. In einem typischen Ausführungsbeispiel der Erfindung arbeitet der Mikrocontroller mit einem Echtzeit-Betriebssystem (660). Gemäß einem alternativen Ausführungsbeispiel weist das Modul einen Mikroprozessor an Stelle des Mikrocontrollers (620) auf.

Gemäß einem weiteren Ausführungsbeispiel der Erfindung besitzt ein Modul, typischerweise das Steuerungsmodul (500), Informationen zur Form, den Abmessungen und dem Fassungsvermögen einer Mehrzahl von unterschiedlichen Probengefäßen, zum Beispiel in Form einer Datenbank. Darin ist eine Anzahl von handelsüblichen Probengefäßen bzw. Probenplatten voreingestellt. Der Typ des Probengefäßes wird durch einen Barcode-Scanner oder einem Laserscanner zur Dimensionserfassung erkannt. Die entsprechenden Parameter zur Form, dem Fassungsvermögen der einzelnen Vertiefungen (Wells) und den Abmessungen der Trägerplatte werden entsprechend der benutzten Trägerplatte für die weitere Bearbeitung durch das Steuerungsmodul berücksichtigt. Auf diese Weise kann ohne manuellen Eingriff der Bedienperson mit verschiedenen Trägerplatten gearbeitet werden. Je nach Typ des Probengefäßes ist lediglich die Verwendung eines entsprechenden Adapters erforderlich, der von der Bedienperson angebracht wird.

In einem weiteren Ausführungsbeispiel der Erfindung werden die Proben aus dem Probenträger nach Eingang in das Gerät durch das Pipettiermodul (260) sukzessive in ein oder mehrere Prozessgefäße umgefüllt, die in den weiteren Verarbeitungsschritten innerhalb der Einheit (120, 140) verwendet werden.

In einem Ausführungsbeispiel der Erfindung ist im Lagerbereich (280) für unbenutzte Hülsen-Anordnungen und die Prozessgefäße ein Behälter vorgesehen, der eine geordnete und Platz sparende Lagerung ermöglicht. Die Verbrauchsmaterialien werden von einer Bedienperson in Behältern über eine Schublade in die Einheit (120) eingeführt. Schienen innerhalb des Behälters dienen als Führung bei der Entnahme durch das Greifermodul (230). Nach Leerung während des Gebrauchs wird der nun leere Behälter vorzugsweise zur Aufbewahrung benutzter Hülsen-Anordnungen und von Prozessgefäßen oder anderen Einwegartikeln benutzt.

Die Hülsen-Anordnungen und die Prozessgefäße sind zur Platz sparenden Lagerung stapelbar. Über die gleiche Schublade werden Pipettenspitzen zugeführt. Über eine Schublade ist der Lagerbereich für weitere zu entsorgende Materialien zugänglich.

Die beschriebene Vorrichtung (100) mit Einheiten und Modulen weist eine Reihe von Vorteilen auf. Automatisierte Geräte zur Probenvorbereitung sind aus einer Vielzahl von Bauteilen aufgebaut und weisen durch das Zusammenspiel der Komponenten einen hohen Komplexitätsgrad auf, der zum Wartungsaufwand und zur Fehleranfälligkeit beiträgt.

Vor dem Hintergrund steigenden Kostendrucks und der daraus folgenden zunehmenden Rationalisierung im Gesundheitswesen steigen jedoch auch die Anforderungen an die Wirtschaftlichkeit von derartigen Laborgeräten. Die bei der vorliegenden Erfindung realisierte hohe Wartungsfreundlichkeit, die einfachen Diagnosemöglichkeiten über das Internet und die durch die Modulbauweise mögliche schnelle Austauschbarkeit von Modulen sind geeignet, zur Senkung der Betriebskosten beizutragen. Damit sinkt die Summe der während der Lebensdauer eines Geräts anfallenden Kosten, auch Total Cost of Ownership (TCO) genannt. Vor allem Wartungskosten und Ausfallzeiten lassen sich durch den beschriebenen Aufbau minimieren, was im Stand der Technik bisher in dieser Form nur wenig berücksichtigt wurde.

Dem Anwender wird zudem eine sehr anwenderfreundliche, nach eigenen Wünschen und Erfordernissen selber zusammenstellbare und kombinierbare Vorrichtung zur Verfügung gestellt. Diese bietet zudem auf Grund der Möglichkeit der durchgehenden Prozessführung von der Primär- oder Sekundärprobe bis zum gewünschten Ergebnis (Tube to Result), ohne dass der Anwender nach dem Start und bis zum Erhalt des Ergebnisses zugegen sein muss, eine deutliche Arbeitserleichterung und ermöglicht eine effiziente Nutzung der Zeit des Anwenders.

## Patentansprüche

1. Vorrichtung (100) zur Probenverarbeitung, insbesondere zur Probenaufbereitung sowie zur Vorbereitung und/oder gegebenenfalls zur Durchführung eines Folgeverfahrens, insbesondere einer Vervielfältigungsreaktion für einen oder mehrere Analyten aus einer biologischen Probe sowie gegebenenfalls zur Durchführung einer Analyse eines oder mehrerer Analyten aus einer biologischen Probe, umfassend
zumindest ein Modul zur Aufnahme und/oder Ausgabe zumindest eines Proben- und/oder Prozessgefäßes,
zumindest ein Modul zum Transport eines oder mehrerer Prozessgefäße, zumindest ein Modul zur Probenaufbereitung, bei dem es sich um ein Lysemodul und/oder um ein Extraktionsmodul handelt, das vorzugsweise eine magnetische Separation durchführt,
zumindest ein Modul zur Vorbereitung eines Folgeverfahrens, insbesondere einer Vervielfältigungsreaktion und/oder eines Analyseverfahrens für einen oder mehrere Analyten,
optional zumindest ein Modul zur Durchführung eines Folgeverfahrens, insbesondere einer Vervielfältigungsreaktion für einen oder mehrere Analyten, optional zumindest ein Modul zur Durchführung einer Analyse eines oder mehrerer Analyten,
wobei die Module auf mindestens zwei Einheiten (120, 140) aufgeteilt sind und das Modul zur Probenaufbereitung in einer ersten Einheit (120) und das Modul zur Vorbereitung eines Folgeverfahrens, insbesondere einer Vervielfältigungsreaktion und/oder eines Analyseverfahrens für einen oder mehrere Analyten, in einer zweiten Einheit (140) angeordnet sind.

2. Vorrichtung (100) zur Probenverarbeitung, insbesondere zur Probenaufbereitung sowie zur Vorbereitung und/oder gegebenenfalls zur Durchführung eines Folgeverfahrens, insbesondere einer Vervielfältigungsreaktion für einen oder mehrere Analyten aus einer Probe sowie gegebenenfalls zur Durchführung einer Analyse eines oder mehrerer Analyten aus einer Probe, umfassend
zumindest ein Modul zur Aufnahme und/oder Ausgabe zumindest eines Proben- und/oder Prozessgefäßes,
zumindest ein Modul zum Transport eines oder mehrerer Prozessgefäße, zumindest ein Modul zur Probenaufbereitung, bei dem es sich um ein Lysemodul und/oder um ein Extraktionsmodul handelt, das vorzugsweise eine magnetische Separation durchführt,
zumindest ein Modul zur Vorbereitung eines Folgeverfahrens, insbesondere einer Vervielfältigungsreaktion und/oder eines Analyseverfahrens für einen oder mehrere Analyten,
optional zumindest ein Modul zur Durchführung eines Folgeverfahrens, insbesondere einer Vervielfältigungsreaktion für einen oder mehrere Analyten, optional zumindest ein Modul zur Durchführung einer Analyse eines oder mehrerer Analyten,
wobei die Module auf zu mindestens zwei Einheiten (120, 140) aufgeteilt sind und die Einheiten jeweils eine eigene Steuerung (500) besitzen, wobei die mindestens zwei Einheiten (120, 140) durch einen ersten Datenbus (400) verbunden sind.

3. Vorrichtung (100) nach Anspruch 1 oder 2, wobei sich die Einheiten (120, 140) jeweils in einem Gehäuse (130, 150) befinden, und die Gehäuse im Wesentlichen baulich getrennt sind.

4. Vorrichtung (100) nach einem der Ansprüche 1 bis 3, wobei vor dem Modul zur Probenaufbereitung ein weiteres Modul zur Probenaufbereitung vorgeschaltet ist, bei dem es sich weder um ein Lyse- noch um ein Extraktionsmodul handelt, sondern vorzugsweise um ein Modul zum physikalischen Zellaufschluss oder zur Entnahme der Probe aus verschlossenen Primär-Probengefäßen, und das bevorzugt in einer weiteren Einheit (110) bereitgestellt ist.

5. Vorrichtung (100) nach einem der vorhergehenden Ansprüche, mit mindestens einem Transfermodul (300) für den Transport eines Prozessgefäßes zwischen zwei Einheiten, wobei das Transfermodul vorzugsweise einen Teleskoparm und/oder ein Förderband aufweist und vorzugsweise bidirektional arbeitet.

6. Vorrichtung (100) nach einem der vorhergehenden Ansprüche, die eine Steuerung umfasst, die spezifische Informationen zu Prozessen und/oder Prozessparametern enthält und die in Abhängigkeit vorab definierter und vor dem Start des Prozesses bestimmter Eigenschaften der Verbrauchsmaterialien und des gewählten Prozesses überprüft, ob der gewählte Prozess durchführbar ist, und wenn erforderlich auf Diskrepanzen hinweist und/oder Möglichkeiten zur Lösung der Diskrepanzen anbietet.

7. Vorrichtung (100) nach einem der vorhergehenden Ansprüche, wobei mindestens eine Einheit, vorzugsweise die Einheit, die das Modul zur Probenaufbereitung umfasst, und/oder die Einheit, die das Modul zur Vorbereitung eines Folgeverfahrens, insbesondere einer Vervielfältigungsreaktion und/oder eines Analyseverfahrens für einen oder mehrere Analyten umfasst, Ausgabe-Prozessgefäße in verschiedenen Ausgangsformaten liefern kann.

8. Vorrichtung (100) nach einem der vorhergehenden Ansprüche, wobei mindestens eine Einheit (110, 120, 140) zur Verbindung mit einem externen Computer (515) angepasst ist, über den Parameter der Vorrichtung (100) eingestellt und abgerufen werden können.

9. Einheit (110, 120, 140) zur Probenverarbeitung, umfassend:
ein Modul zur Aufnahme und/oder Ausgabe eines oder mehrerer Proben- und/oder Prozessgefäße und ein oder mehrere identische oder voneinander verschiedene weitere Module ausgewählt aus der Gruppe umfassend
a) ein Modul zum Transport eines oder mehrerer Prozessgefäße innerhalb der Einheit,
b) ein Transfermodul (300) für den Transport eines oder mehrerer Prozessgefäße zwischen zwei Einheiten, wobei das Transfermodul vorzugsweise einen Teleskoparm und/oder ein Förderband aufweist und vorzugsweise bidirektional arbeitet,
c) ein Modul zur Probenaufbereitung, bei dem es sich um ein Lysemodul und/oder ein Extraktionsmodul, welches vorzugsweise eine magnetische Separation durchführt, handelt,
d) ein Modul zur Vorbereitung eines Folgeverfahrens, insbesondere einer Vervielfältigungsreaktion und/oder eines Analyseverfahrens, bei dem es sich vorzugsweise um ein Pipettiermodul und/oder ein Kühlmodul handelt,
e) ein Modul zur Durchführung eines Folgeverfahrens, insbesondere einer Vervielfältigung und/oder zur Analyse eines oder mehrerer Analyten und
f) ein Modul zur Aufbewahrung der verarbeiteten Proben, insbesondere der aufbereiteten Proben,
g) ein von (c) verschiedenes Modul zur Probenaufbereitung, bei dem es sich vorzugsweise um ein Modul zum physikalischen Zellaufschluss oder zur Entnahme der Probe aus verschlossenen Primär-Probengefäßen handelt,
wobei sich die Module (c) und (d) nicht in einer Einheit befinden und wobei die Einheit eine Steuerung (500) aufweist und mit einer zweiten Einheit über einen Datenbus (400) kommuniziert, und wobei die Module innerhalb der Einheit über einen zweiten Datenbus (670) kommunizieren.

10. Einheit (110, 120, 140) nach Anspruch 9, wobei die Einheit ein im Wesentlichen abgeschlossenes Gehäuse (130, 150) aufweist.

11. Vorrichtung (100) nach einem der Ansprüche 1 bis 8, oder Einheit (110, 120, 140) nach einem der Ansprüche 9 oder 10, wobei mindestens eine der Einheiten (110, 120, 140) je einen oder mehrere Bereiche (280, 290) zur Lagerung von benutzten und/oder unbenutzten Verbrauchsmaterialien aufweist, wobei der oder die Bereiche zur Lagerung der unbenutzten Verbrauchsmaterialien vorzugsweise räumlich von dem oder den Bereichen zur Lagerung der benutzten Verbrauchsmaterialien getrennt ist (sind).

12. Vorrichtung (100) nach einem der Ansprüche 1 bis 8 oder 11 oder Einheit (110, 120, 140) nach einem der Ansprüche 9 bis 11, die eine Steuerung umfassen, die spezifische Informationen zu verschiedenen Arten von Verbrauchsmaterialien aufweist, vorzugsweise zu deren benötigter Menge für einen geplanten Prozessablauf sowie zu der Form, den Abmessungen, dem Fassungsvermögen und dem Inhalt von Proben-, Prozess- und/oder Vorratsgefäßen, und wobei diese Informationen nach Bestimmung einer oder mehrerer vorab definierter Eigenschaften der Verbrauchsmaterialien vor Beginn eines Prozessablaufs, vorzugsweise bei Beladung der Vorrichtung (100), durch eine Sensorik, vorzugsweise durch einen Lasersensor und/oder eine Digitalkamera und/oder einen Ultraschallsensor für den Prozessverlauf genutzt werden.

13. Vorrichtung nach einem der Ansprüche 1 bis 8 oder 11 bis 12 oder Einheit (110, 120, 140) nach einem der Ansprüche 9 bis 12, umfassend ein Modul zur Aufnahme von Probengefäßen, das mittels Adaptern zur Aufnahme einer Mehrzahl von unterschiedlichen Typen von Probengefäßen anpassbar ist.

14. Vorrichtung (100) nach einem der Ansprüche 1 bis 8 oder 11 bis 13 oder Einheit (120, 140) nach einem der Ansprüche 9 bis 13, wobei während eines Prozessdurchlaufs zur Verarbeitung einer Probe
a) in der Einheit, die das Modul zur Probenaufbereitung umfasst, mindestens 2, vorzugsweise geometrisch identische, Prozessgefäße und vorzugsweise mindestens ein weiteres geometrisch von diesen verschiedenes Probengefäß zum Einsatz kommen
b) in der Einheit, die das Modul zur Vorbereitung eines Folgeverfahrens, insbesondere einer Vervielfältigungsreaktion und/oder eines Analyseverfahrens umfasst, ein Prozessgefäß, und ein oder mehrere weitere Prozess- und/oder Vorratsgefäße, die unabhängig voneinander untereinander und zum ersten Prozessgefäß geometrisch identisch oder verschieden sein können, zum Einsatz kommen,
wobei es sich bei dem Probengefäß weder um ein Prozess- noch um ein Vorratsgefäß handelt.

15. Modul zur Verwendung in einer Einheit (110, 120, 140) zur biologischen Probenverarbeitung, umfassend:
a) eine Halterung für mindestens ein Gefäß,
b) einen Mikrocontroller (620),
c) eine Datenbus-Schnittstelle zur Daten-Kommunikation mit der Steuerung der Einheit.

16. Modul nach Anspruch 15, mit einer Transfereinrichtung für den Transport eines Proben- und/oder Prozessgefäßes zwischen zwei Einheiten, wobei das Transfermodul vorzugsweise einen Teleskoparm und/oder ein Förderband aufweist und vorzugsweise bidirektional arbeitet.

17. Modul nach einem der Anspruch 15 oder 16, wobei der Mikrocontroller (620) mit einem Echtzeit-Betriebssystem (660) arbeitet.

18. Modul nach einem der vorhergehenden Ansprüche, ferner umfassend einen Festwertspeicher und/oder einen Arbeitsspeicher und/oder einen elektronischen Schaltkreis zur Kommunikationssteuerung.
